Europäisches Patentamt

European Patent Office

Office européen des brevets

(19)

(11) Veröffentlichungsnummer: **0 229 971**
**B1**

# (12) EUROPÄISCHE PATENTSCHRIFT

(45) Veröffentlichungstag der Patentschrift: **07.11.90**

(51) Int. Cl.⁵: **A 61 B 6/14, A 61 B 6/00**

(21) Anmeldenummer: **86117087.6**

(22) Anmeldetag: **08.12.86**

(54) **Zahnärztliches Röntgendiagnostikgerät zur Erstellung von Panorama-Schichtaufnahmen vom Kiefer eines Patienten.**

(30) Priorität: **20.12.85 DE 3545509**

(43) Veröffentlichungstag der Anmeldung:
**29.07.87 Patentblatt 87/31**

(45) Bekanntmachung des Hinweises auf die
Patenterteilung:
**07.11.90 Patentblatt 90/45**

(84) Benannte Vertragsstaaten:
**DE FR GB IT**

(56) Entgegenhaltungen:
**DE-A-2 461 441**
**DE-A-2 630 135**

**ELECTRO MEDICA SIEMENS, Band 46, Nr. 4, 1978, Seiten 133-135, Erlangen, DE; A. ULBRICHT: "Die Bewährung des Röntgenschichtaufnahmegerätes Optiplanimat in der täglichen Routine"**

(73) Patentinhaber: **Siemens Aktiengesellschaft**
**Wittelsbacherplatz 2**
**D-8000 München 2 (DE)**

(72) Erfinder: **Heubeck, Erich**
**Blütenweg 11**
**D-6140 Bensheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung bezieht sich auf ein zahnärztliches Röntgendiagnostikgerät zur Erstellung von Panorama-Schichtaufnahmen vom Kiefer eines Patienten, enthaltend eine an einem Laufwagen eines Stativs angeordnete Dreheinheit mit einem Träger, an dem eine Strahlenquelle und diametral dazu eine Halterung für eine Filmkassette derart verstellbar gehalten sind, daß von der Strahlenquelle ausgehende Strahlen auf die Filmkassette im wesentlichen senkrecht auftreffen, enthaltend ferner eine Verstelleinrichtung, mit welcher der Träger in einer dem Zahnbogen entsprechenden Kurve so verstellbar ist, daß die Zähne aufeinanderfolgend mit dem Kiefer auf dem Film abbildbar sind.

Solch eine Röntgendiagnostikeinrichtung ist aus DE—A—2630135 bekannt. Mit bekannten solchen Röntgendiagnostikgeräten (Prospekt M-D 80/1361 ORTHOPANTHOMOGRAPH 10) sind derartige Aufnahmen nur in einer dem Gerät fest zugeordneten Schichtlage möglich. Damit für Standard- bzw. Routineuntersuchungen ein relativ großer Diagnosebereich erfaßt wird, ist der Aufnahmeablauf so gewählt, daß sich eine möglichst große Schichtdicke ergibt. Durch die große Schichtdicke kann es jedoch bei hintereinanderliegenden Objektdetails zu überlagerten Abbildungen kommen. Diese führt letztlich dazu, daß einzelne Objekte teils nicht bzw. nicht genau diagnostiziert werden können.

Der im Anspruch 1 angegebenen Erfindung liegt die Aufgabe zugrunde, ein Röntgendiagnostikgerät der vorgenannten Gattung dahingehend zu verbessern, daß mit dem Gerät auch Aufnahmen in mehreren Schichtlagen auf einem Film möglich sind.

Wenngleich es aus der allgemeinen Röntgentechnik unter dem Begriff "Simultanschichtverfahren" bekannt ist, mehrere parallele Körperschichten gleichzeitig in Röntgenbildern darzustellen (DE—A—12 44 555), so lassen sich die dort vorgesehenen Konstruktionen für Panorama-Aufnahmen nicht anwenden, u.a. deswegen nicht, weil bei dem bekannten "Simultanschichtverfahren" eine Vielzahl von Filmen mit Verstärkerfolien (Filmfoliensätze) übereinandergestapelt in einer Kassette angeordnet sind und die einzelnen Foliensätze während der Aufnahme parallel zu sich selbst verschoben werden müssen.

Ein wesentlicher Vorteil des erfindungsgemäßen Gerätes ist, daß auf einem Film von einem (oder mehreren) beliebig wählbaren Abschnitt(en) des Kiefers Abbildungen in mehreren Schichtlagen nebeneinander gemacht werden können.

Ein Ausführungsbeispiel der Erfindung wird nachfolgend anhand der Zeichnung näher erläutert.

Es zeigen:

Figur 1 eine Ausführungsform des erfindungsgemäßen Gerätes in einer schaubildlichen Darstellung,

Figur 2 das Gerät von oben gesehen mit vereinfachter Darstellung der Verstellmechanik,

Figur 3 einen Kieferbogen mit vereinfachter Darstellung der erfindungsgemäßen Multischichtung,

Figur 4 eine vereinfachte Darstellung eines Teiles eines Röntgenfilmes mit Abbildungen in verschiedenen Schichtlagen,

Figur 5 ein Blockschaltbild zur Erläuterung der Antriebe.

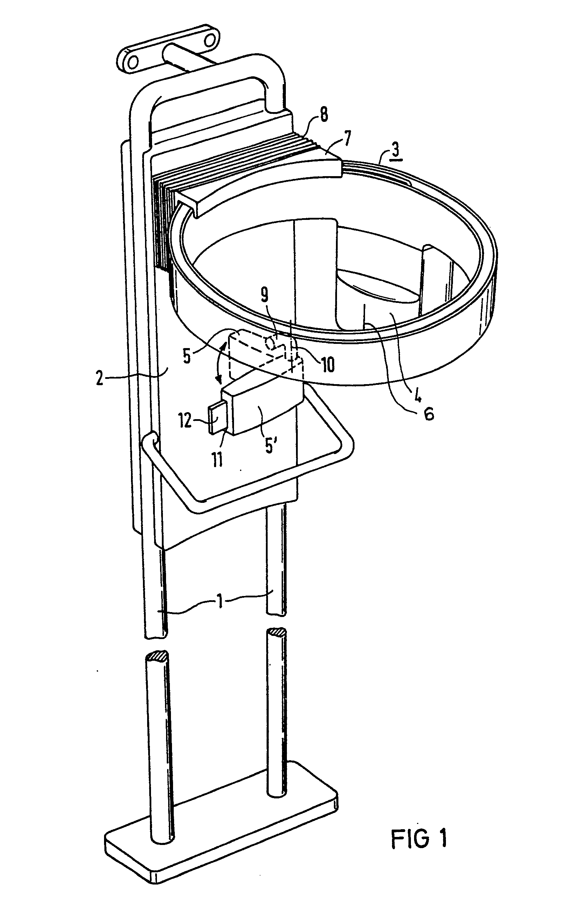

Die Figur 1 zeigt in einer schaubildlichen Darstellung eine Ausführungsform eines zahnärztlichen Röntgendiagnostikgerätes gemäß der Erfindung. Das Gerät enthält ein aus zwei Standrohren gebildetes Stativ 1, an dem ein Laufwagen 2 höhenverstellbar gehalten ist. Am Laufwagen 2 ist eine Dreheinheit in Form eines in sich geschlossenen Ringes gehalten, die einerseits eine Röntgenstrahlenquelle 4 und diametral dazu eine Filmkassettenhalterung 5 enthält. Während die Strahlenquelle 4, deren Strahlenaustrittsöffnung mit 6 bezeichnet ist, am Drehring 3 drehfest angeordnet ist, ist der Filmkassettenhalter 5 mittels vertikaler Achslagerung 10 an einem abgewinkelten Tragarm 9 in Pfeilrichtung schwenkbar gehalten. Der Filmkassettenhalter 5 kann so aus der gestrichelt gezeichneten Gebrauchsstellung, in die mit durchgehenden Linien gezeichnete Nichtgebrauchslage gebracht werden, die einerseits der Helferin das Positionieren des Patientenkopfes erleichtert und andererseits das Erstellen von Fernaufnahmen, sog. Ceph-Aufnahmen, ermöglicht, für welche bei bisherigen Geräteausführungen die Röntgenstrahlenquelle gedreht werdenmußte.

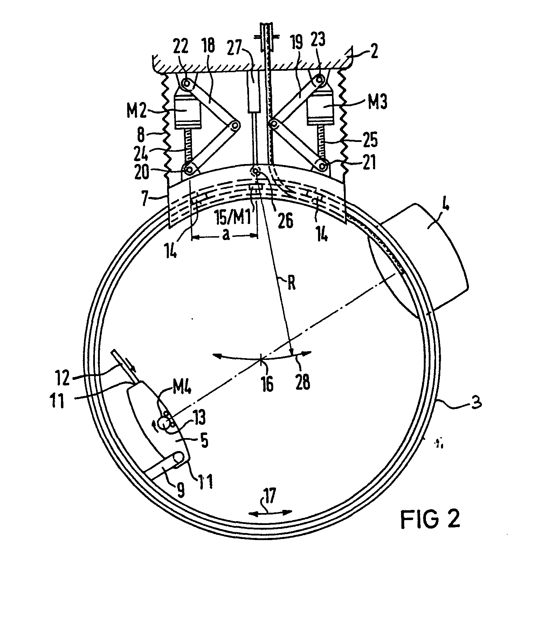

Der Filmkassettenhalter 5 enthält an beiden Stirnseiten schlitzförmige Ein- und Austrittsöffnungen 11, über die die mit 12 bezeichnete Filmkassette eingeführt bzw. nach der Aufnahme entnommen werden kann. Bei der verwendeten Filmkassette handelt es sich um eine flexible, mit Verstärkerfolie versehene Filmkassette, wie sie im Prinzip für intraorale Aufnahmen verwendet werden. Der Transport der Filmkassette erfolgt mittels eines in Figur 2 allgemein mit M4 bezeichneten elektromotorischen Antriebes.

Der Drehring 3 ist in einem Lagerteil 7 drehbar und gegenüber dem Laufwagen 2 auch schwenkbar gehalten. Die in Figur 2 näher erläuterte Verstellmechanik zwischen Laufwagen 2 und Drehring 3 ist durch einen Faltenbalg 8 abgedeckt.

Zur Verstellung des Drehringes 3 sind zwei Scherenarme 18, 19 vorgesehen, deren eine Enden an den mit 20, 21 bezeichneten Gelenkstellen am Lagerteil 7 und deren andere Enden an den mit 22 und 23 bezeichneten Gelenkstellen am Laufwagen 2 angelenkt sind. Zwischen den Gelenkstellen 20, 22 einerseits und 21, 23 andererseits sind elektromotorisch angetriebene Spindelantriebe 24, 35 vorgesehen, deren Antriebe M2, M3 über eine Steuereinheit individuell angesteuert werden können. Mittig zwischen den beiden Gelenkstellen 20, 21 ist in einem Abstand a am Lagerteil 7 ein Schwenklager 26 vorgesehen, an dem das eine Ende eines Teleskoparmes (27) angelenkt ist, dessen anderes Ende starr am Laufwagen 2 befestigt ist.

In Verbindung mit der vorbeschriebenen Scherenarmkonstruktion ist es möglich, durch entsprechende Steuerung der beiden Antriebe M2, M3 den Drehring und damit die Position von Röntgenstrahlenquelle 4 und Filmkassettenhalter 5 in jede beliebige, für den Umlauf um den Patientenkopf notwendige Position zu bringen. Bei gleich schnellem Antribs beider Antriebe M1, M2 kann der Drehring parallel zum Laufwagen 2 verstellt werden, bei nicht gleichmäßiger Verstellung der beiden Antriebe um die Schwenkachse 26 so verschwenkt werden, daß der Mittelpunkt 16 des Drehringes 3 eine Querbewegung (von etwa ±40 mm) mit einem Radius R von ca. 350 mm in der mit 28 bezeichneten Pfeilrichtung ausführt.

In Verbindung mit der Eigendrehbewegung, die der Drehring 3 auch noch um seine Mittelpunktsachse 16 ausführen kann, wozu ein weiterer, in der Figur mit M1 bezeichneter Antrieb vorgesehen ist, dessen Antriebs- und Führungsrollen mit (14, 15) bezeichnet sind, kann so der Bewegungsablauf, welcher bei bekannten Geräten dieser Gattung nur mittels aufwendiger Mechanik erzielt wird, auf relativ einfache Weise nachvollzogen werden.

Die erwähnten Antriebs- und Führungsrollen (14, 15) sind an Ober- und Unterseite des Drehringes 3 so angeordnet, daß sie eine Dreieckslagerung bilden. Zweckmäßigerweise ist an der Oberseite in der Mitte eine mit dem Antriebsmotor M1 gekuppelte Antriebsrolle und beidseitig davon an der Unterseite je eine Führungsrolle vorgesehen. Das Lagerteil 7 ist hierzu entsprechend winkelig ausgebildet.

Der Filmkassettenhalter 5 besteht aus zwei durch Längsteilung gebildeten Halbschalen, die durch eine leicht lösbare Schraubverbindung miteinander verbunden sind, wodurch im Servicefalle ein rascher Zugang zum Antrieb M4 gegeben ist. Der Antrieb M4 für den Durchlauf der Filmkassette kann, ebenso wie die Antriebe M1 bis M3, über eine in Figur 5 mit 30 bezeichnete Steuereinheit individuell angesteuert werden.

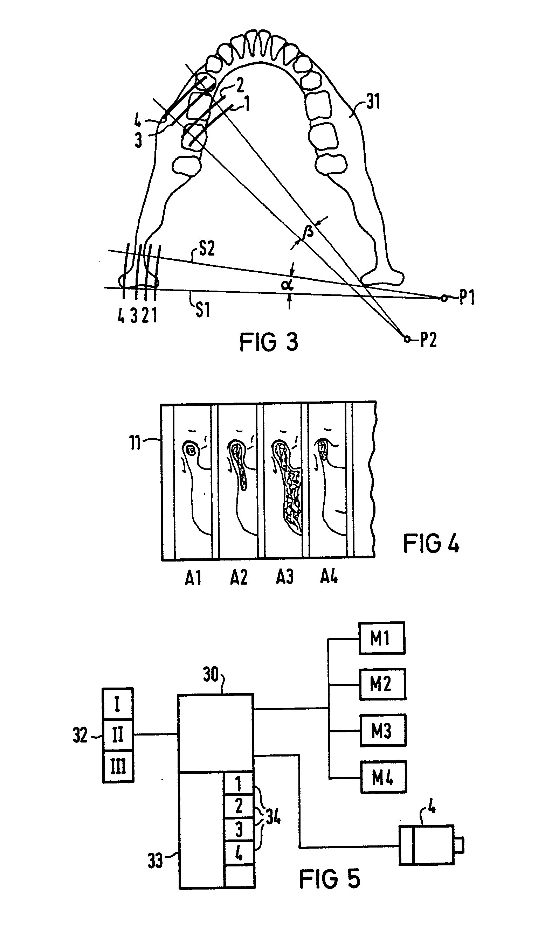

Bevor der Ablauf näher erläutert wird, seien anhand der Figuren 3 und 4 die mit dem erfindungsgemäßen Gerät zu erstellenden Multischichtaufnahmen näher erläutert. In Figur 3 ist hierzu schematisch ein Kieferbogen 31 in der Draufsicht dargestellt. Es wird angenommen, daß zunächst vier verschiedene Schichtaufnahmen vom linken Kiefergelenk und danach vier weitere Schichtaufnahmen von einem weiteren Abschnitt des Kiefers erstellt werden sollen.

Die Dreheinheit 3 wird zunächst mittels der Verstellantriebe M1, M2, M3 in eine Position P1 gefahren, von der aus von der Strahlenquelle 4 Strahlen auf den gewünschten Abbildungsbereich abgegeben werden können, der beispielsweise den eingezeichneten Winkel α umfassen soll. Die Position P1, die die Dreheinheit 3 einnimmt, ist durch in einem Programmspeicher 32 abgelegte Größen definiert. Das gleiche gilt für weitere Ausgangspositionen P2 ... zur Aufnahme weiterer Abschnitte, für die ebenfalls im Programmspeicher 32 entsprechende Größen abgelegt sind. Bei Betätigen der Programmtaste I werden über die Steuereinheit 30 den Verstellmotoren M1 bis M3 entsprechende Signale gegeben, wodurch die Dreheinheit in die erste Ausgangsposition P1 fährt. Entsprechendes gilt für die weiteren Positionen, die durch Betätigen der Programmtasten II, III ... angefahren werden können.

Die Steuereinheit 30 ist mit einem weiteren Speicher 33 verbunden, in dem entsprechend der Anzwahl der vorgesehenen Schichtlagen, im vorliegenden Ausführungsbeispiel also vier, entsprechende Speichergrößen 33/1 bis 33/4 abgespeichert sind. Diese Speichergrößen entsprechen verschiedenen Drehzahlen, mit denen der Antriebsmotor M4 betrieben und damit die Filmkassette 12 an der Sekundärschlitzblende im Filmkassettenhalter 5 vorbeibewegt wird. Davon ausgehend, daß die Schichtlage von der Geschwindigkeit abhängig ist, mit der die Filmkassette gegenüber dem auftreffenden Zentralstrahl an der Sekundärblende vorbeigeführt wird, sind also im Speicher 33 verschiedene, den gewünschten Schichtlagen entsprechende Speichergrößen abgelegt. Diese Speichergrößen können durch Schichtlagen-Programmtasten 34 abgerufen werden.

Wird beispielsweise, ausgehend von der Ausgangsposition P1, entsprechend dem vorgewählten Programm I eine Aufnahme vom linken Kiefergelenk in der mit 1 in Figur 3 bezeichneten Schichtlage 1 gewünscht, so wird diese Schichtlage durch die Speichertaste 34/1 vorgewählt. Die entsprechende Größe wird der Steuereinheit 30 übermittelt, die dann den Antriebsmotor 4 mit einer der Schichtlage 1 entsprechenden Geschwindigkeit am Zentralstrahl vorbeibewegt. Nachdem die Aufnahme in dem dem Winkel α entsprechenden Abbildungsabschnitt in dieser Schichtlage 1 erstellt ist, wird die Strahlenquelle 4 abgeschaltet und die Dreheinheit um den Schwenkwinkel α wieder zurück in die Ausgangsposition gefahren. Danach kann entweder individuell durch erneutes Drücken einer weiteren Schichtwahltaste 34 oder automatisch die nächste Aufnahme in einer anderen Schichtlage (2, 3 oder 4) erstellt werden. Die Filmkassette 11 wird während dieser wiederholten Aufnahme weitertransportiert, so daß auf dem Film die Objektdetails in den verschiedenen Schichtlagen nebeneinander abgebildet werden.

Sofern es die Filmlänge aufgrund des gewählten Abbildungsausschnittes (Winkel α) erlaubt, noch weitere Objektausschnitte darzustellen, kann dieser Vorgang von einer beliebigen anderen Stelle, beispielsweise vom Punkt P2 aus, wiederholt werden, d.h. auch hier wird entsprechend dem gewählten Objektausschnitt (Winkel β) und dem gewählten Programm der gewählte Objektausschnitt in mehreren Schichten (1 bis 4) auf dem Film nebeneinander abgebildet. Auch diese unterschiedlichen Schichtlagen sind durch unterschiedliche Drehzahlen des Antriebsmotors 4 definiert, die im Speicher 33 abgelegt sind und durch Schichtlagenvorwahltasten 34 abgerufen werden können.

## Patentansprüche

1. Zahnärztliches Röntgendiagnostikgerät zur Erstellung von Panorama-Schichtaufnahmen vom Kiefer eines Patienten, enthaltend eine an einem Laufwagen (2) eines Stativs (1) angeordnete Dreheinheit mit einem Träger (3) an dem eine Strahlenquelle (4) und diametral dazu eine Halterung (5) für eine Filmkassette (12) derart verstellbar gehaltert sind, daß von der Strahlenquelle ausgehende Strahlen auf die Filmkassette im wesentlichen senkrecht auftreffen, enthaltend ferner eine Verstelleinrichtung, mit welcher der Träger in einer dem Zahnbogen entsprechenden Umlaufkurve verstellbar ist, dergestalt, daß die Zähne aufeinanderfolgend mit dem Kiefer auf dem Film abgebildet werden, wobei die Verstelleinrichtung erste Verstellmittel (30—34) enthält, mit welchen der Träger (3) um eine erste vertikale Achslagerung (26) gedreht werden kann, dadurch gekennzeichnet, daß zweite Verstellmittel (17—24) vorhanden sind, mit welchen der Träger (3) während der Drehbewegung um die erste Achslagerung eine Schwenkbewegung quer zur Symmetrieachse des Objektes ausführt, wobei der Schwenkradius (R) und die Auslenkung (c) der Schwenkbewegung so gewählt sind, daß bei der Bewegung der Dreheinheit (3) stets eine senkrechte Durchstrahlrichtung durch das Objekt bei im wesentlichen konstantem Abstand (d) von Objekt und Film vorgesehen ist, daß ferner die Filmkassette (12) unabhängig von den Verstellmitteln für die Dreheinheit (3) relativ zur Strahlenquelle (4) verstellbar angeordnet ist, und daß eine Steuereinheit (30) vorgesehen ist, welche

a) die Dreheinheit (3) so steuert, daß sie von einer Ausgangsposition (P1, P2) aus nacheinander mehrmals um einen, einem Objektabschnitt entsprechenden Winkelbereich (α;β) verstellt wird, wobei dabei die Strahlenquelle (4) zu Beginn der Verstellung eingeschaltet und nach Erreichen des Verstellwinkels (α,β) wieder abgeschaltet wird,

b) den Filmkassettenantrieb (M4) in jeder Wiederholphase der Durchstrahlung des Objektabschnittes mit einer einer gewählten Schichtlage (1 bis 4) entsprechenden Geschwindigkeit steuert,

c) nach jeder Wiederholphase der Durchstrahlung des Objektabschnittes die Filmkassette (12) um einen Abbildungsabschnitt (A1, A2 in Fig. 4) gegenüber der Strahlenquelle (4) weitertransportiert.

2. Röntgendiagnostikgerät nach Anspruch 1, dadurch gekennzeichnet, daß mit der Steuereinheit (30) ein vorzugsweise elektronischer Speicher (33) verbunden ist, in dem die den gewünschten Schichtlagen (1 bis 4) entsprechenden Steuersignale abgespeichert sind.

3. Röntgendiagnostikgerät nach Anspruch 2, dadurch gekennzeichnet, daß Schichtlagen-Vorwahltasten (34) vorgesehen sind, bei deren Betätigung die im Speicher (33) abgelegten Speicherwerte abgerufen werden.

4. Röntgendiagnostikgerät nach Anspruch 1, dadurch gekennzeichnet, daß die Positionen verschiedene Objektabschnitte in einem Programm-Speicher (32) abgelegt und durch Programmtasten (I, II ... N) abrufbar sind.

5. Röntgendiagnostikgerät nach Anspruch 2, dadurch gekennzeichnet, daß als Antrieb (M4) für die Verstellung der Filmkassette (12) ein Schrittmotor vorgesehen ist, dessen Drehzahl über die Steuersignale aus dem Speicher (33) bestimmt wird.

## Revendications

1. Appareil de radiodiagnostic dentaire pour réaliser des tomographies panoramiques de la mâchoire d'un patient, comportant une unité rotative disposée sur un chariot (2) d'un statif (1) et comportant un support (3), sur lequel sont disposés une source de rayonnement (4) et, en position diamétralement opposée de cette dernière, un support (5) pour une cassette à film (12), qui peut être déplacée de telle sorte que des moyens émis par une source de rayonnement tombent sensiblement perpendiculairement sur la cassette à film, et comportant en outre un dispositif de réglage à l'aide duquel on peut déplacer le support sur une courbe circonférentielle correspondant à l'arc dentaire de telle sorte que l'image des dents, ainsi que la mâchoire, est formée successivement sur le film, et dans lequel le dispositif de réglage contient des premiers moyens de réglage (30—34), à l'aide desquels le support (3) peut être entraîné en rotation autour d'un premier support d'axe vertical (26), caractérisé par le fait qu'il est prévu des seconds moyens de réglage (17—24), à l'aide desquels le support (3) exécute, pendant le mouvement de rotation autour du premier support d'axe, un mouvement de pivotement transversalement par rapport à l'axe de symétrie de l'objet, le rayon de pivotement (R) et le débattement (c) du mouvement étant choisis de manière que lors du déplacement de l'unité rotative (3), on ait en permanence une direction verticale d'irradiation à travers l'objet pour une distance d sensiblement constante entre l'objet et le film, qu'en outre la cassette à film (12) est disposée de manière à être réglable, indépendamment des moyens de réglage pour l'unité rotative (3), par rapport à la source de rayonnement (4) et qu'il est prévu une unité de commande (30), qui

a) commande l'unité rotative (3) de manière qu'elle soit déplacée depuis une position initiale (P1, P2) successivement, à plusieurs reprises, sur une plage angulaire (α;β) correspondant à une section de l'objet, la source de rayonnement (4) étant branchée au début du réglage et étant à nouveau débranchée une fois que l'angle de déplacement (α,β) a été atteint, et

b) commande le dispositif (M4) d'entraînement de la cassette à film lors de chaque phase de répétition de l'irradiation de la section de l'objet, avec une vitesse correspondant à une position de couche sélectionnée (1 à 4), et

c) continue à entraîner la cassette à film (12) sur une section de formation d'images (A1, A2 sur la figure 4) par rapport à la source de rayonnement (4), après chaque phase de répétition de l'irradiation de la section de l'objet.

2. Appareil de radiodiagnostic suivant la revendication 1, caractérisé par le fait qu'à l'unité de commande (30) est reliée une mémoire (33), de préférence électronique, dans laquelle les signaux de commande correspondant aux positions de couches désirées (1 à 4) sont mémorisés.

3. Appareil de radiodiagnostic suivant la revendication 2, caractérisé par le fait qu'il est prévu des touches (34) de présélection des positions de couches, lors de l'actionnement desquelles les valeurs mémorisées dans la mémoire (33) sont appelées.

4. Appareil de radiodiagnostic suivant la revendication 1, caractérisé par le fait les positions de différentes sections de l'objet sont mémorisées dans une mémoire de programme (32) et peuvent être appelées à l'aide de touches de programme I, II ... (N).

5. Appareil de radiodiagnostic suivant la revendication 2, caractérisé par le fait qu'il est prévu, comme dispositif d'entraînement (M4) pour le déplacement de la cassette à film (12), un moteur pas-à-pas, dont la vitesse de rotation est déterminée à partir de la mémoire (33) par l'intermédiaire des signaux de commande.

## Claims

1. Dental X-ray diagnostic apparatus for preparing panoramic tomographic exposures of the jaw of a patient, containing a rotating unit arranged on a carriage (2) of a stand (1) and having a support (3) on which a radiation source (4) and, diametrically thereto, a holder (5) for a film cassette (12) are held in an adjustable manner in such a way that rays emanating from the radiation source strike the film cassette substantially perpendicularly, the apparatus containing moreover an adjusting device with which the support can be adjusted in a circular curve corresponding to the arc of the teeth, in such a way that the teeth are imaged successively with the jaw on the film, with the adjusting device containing first adjusting means (30—34) with which the support (3) can be rotated about a first vertical axle mounting (26), characterized in that second adjusting means (17—24) is present with which the support (3) during rotation about the first axle mounting carries out a rotation at right-angles to the axis of symmetry of the object, whereby the radius (R) of rotation and the deflection (c) of the rotation are chosen such that with the movement of the rotating unit (3) there is always provided a perpendicular irradiation direction through the object with substantially constant distance (d) from object and film, in that moreover the film cassette (12) is arranged in an adjustable manner independent of the adjusting means for the rotating unit (3) and relative to the radiation source (4), and in that a control unit (30) is provided which

a) controls the rotating unit (3) in such a way that it is adjusted from an output position (P1, P2) several times one after the other around an angle region (α;β) corresponding to an object section, the radiation source (4) then being switched on at the beginning of the adjustment and being switched off again after reaching the adjusting angle (α,β),

b) controls the film cassette drive (M4) in each repeat phase of the irradiation of the object section at a speed corresponding to a chosen layer position (1 to 4),

c) after every repeat phase of the irradiation of the object section transports the film cassette (12) further around an image section (A1, A2 in Figure 4) relative to the radiation source (4).

2. X-ray diagnostic apparatus according to Claim 1, characterized in that there is connected to the control unit (30) a memory (33), preferably electronic, in which the control signals corresponding to the desired layer positions (1—4) are stored.

3. X-ray diagnostic apparatus according to Claim 2, characterized in that layer position preselection keys (34) are provided, on whose actuation the stored values deposited in the memory (33) are recalled.

4. X-ray diagnostic apparatus according to Claim 1, characterized in that the positions of various object sections are deposited in a program memory (32) and can be recalled by program keys (I, II ... N).

5. X-ray diagnostic apparatus according to Claim 2, characterized in that a stepping motor is provided as drive (M4) for the adjustment of the film cassette (12), the rotational speed of which motor is determined by means of the control signals from the memory (33).

FIG 1

EP  0 229 971  B1

FIG 2

2

FIG 3

FIG 4

A1    A2    A3    A4

FIG 5